# FASCICULE DE BREVET EUROPEEN

(11) **EP 1 441 704 B1**
(45) Date de publication et mention de la délivrance du brevet: **26.01.2005**
(21) Numéro de dépôt: 02796830.4
(22) Date de dépôt: 28.10.2002
(51) Int. Cl.: A61K 9/26, A61K 9/50

(54) **COMPRIME ORODISPERSIBLE PRESENTANT UNE GRANDE HOMOGENEITE ET SON PROCEDE DE PREPARATION**
ORODISPERSIBLE TABLETTE MIT HOHER HOMOGENITÄT UND HERSTELLUNGSVERFAHREN DAFÜR
ORODISPERSIBLE TABLET HAVING HIGH HOMOGENEITY AND THE PREPARATION METHOD THEREOF

(30) Priorité: 05.11.2001 FR 0114269
(43) Date de publication de la demande: 04.08.2004
(73) Titulaire: ETHYPHARM, 78550 Houdan (FR)
(72) Inventeur: GENDROT, Edouard, F-28500 Garnay (FR); NOURI, Nourredine, F-06400 Cannes (FR); SUPLIE, Pascal, F-27400 Montaure (FR)
(74) Mandataire: Nargolwalla, Cyra
(86) Numéro de dépôt international: PCT/FR2002/003690
(87) Numéro de publication internationale: WO 2003/039520

(56) Documents cités:
- EP-A- 0 745 382
- WO-A-81/02671
- FR-A- 2 784 895
- FR-A- 2 790 387

## Description

La présente invention a pour objet un comprimé orodispersible, c'est à dire un comprimé à dispersion rapide du genre de ceux qui se désagrègent dans la bouche en moins de 40 secondes et même en moins de 30 secondes. Elle vise également le procédé de préparation de ce comprimé.

Les comprimés à délitement rapide déjà connus par exemple ceux décrits par la Société Prographarm dans FR9109245, FR9208642, FR9709233, FR9814034, donnent satisfaction mais pourraient être améliorés du point de vue de l'homogénéité de la répartition de la matière active au sein du comprimé et d'un comprimé à l'autre, particulièrement lorsque le dosage unitaire de ce dernier est faible ou modéré, représentant par exemple moins de 25% de la masse unitaire du comprimé. Il arrive que des hétérogénéités de répartition de la ou des matières actives au sein du comprimé se présentent, pouvant entraîner des modifications de caractère physico-chimique des comprimés.

L'invention a pour but, surtout, de remédier à ces inconvénients et de fournir des comprimés présentant une grande homogénéité de la répartition en matière active au sein du comprimé et d'un comprimé à l'autre, tout en maintenant une abrasion (mesurée comme indiqué dans la pharmacopée française (Xème édition, "V.5.1- abrasion des comprimés", janvier 1993)) inférieure à 2%, et présentant un excellent temps de délitement au contact de la bouche et une palatabilité agréable, caractéristiques essentielles des comprimés décrits précédemment par la Société Demanderesse.

De façon surprenante et inattendue, la Société Demanderesse a trouvé que toutes ces propriétés pouvaient être réunies dans un comprimé comprenant des microcristaux ou microgranules de matière active enrobée et un mélange d'excipients se présentant sous la forme de grains, en choisissant un mélange approprié d'excipients et en respectant un rapport de granulométrie précis entre les microgranules et les grains d'excipient.

Ainsi, la présente invention porte sur des comprimés à délitement rapide du genre de ceux qui sont destinés à se désintégrer dans la bouche au contact de la salive en moins de 40 secondes, de préférence en moins de 30 secondes, et en formant une suspension facile à avaler, qui sont à base d'au moins une matière active sous forme de microcristaux ou de microgranules enrobés et d'un mélange d'excipients sous forme de grains, le mélange d'excipients comprenant notamment:
de 60 à 85% d'un diluant,
de 3% à 20% d'un agent de désagrégation,
de 1% à 8% d'un édulcorant,
de 0% à 5% d'un agent d'écoulement,
de 0% à 10% d'un liant,
de 0% à 10% d'un agent perméabilisant, d'un agent gonflant et/ou d'un agent lubrifiant,
les pourcentages étant des pourcentages en poids par rapport au poids total des grains d'excipients,
et les grains d'excipients ayant une granulométrie médiane comprise entre +30 et -30%, de préférence entre +10 et -10%, par rapport à la dimension des microcristaux ou microgranules enrobés.

Dans un mode de réalisation avantageux de l'invention, le mélange d'excipients peut en outre comprendre au moins un agent aromatisant et/ou au moins un colorant.

Le temps de délitement dans la bouche correspond à la durée qui sépare, d'une part, le moment de la mise en place du comprimé dans la bouche au contact de la salive et, d'autre part, le moment de la déglutition de la suspension résultant de la désagrégation du comprimé au contact de la salive.

Le mélange d'excipients sous forme de grains entrant dans la constitution des comprimés conformes à l'invention comprend un diluant, un agent de désagrégation, un édulcorant, un agent d'écoulement, et éventuellement d'autres excipients choisis dans le groupe comprenant notamment un liant, un agent gonflant, un agent lubrifiant, un colorant et un agent aromatisant.

Le diluant entrant dans la constitution des grains d'excipients est choisi dans le groupe comprenant notamment les polyols de moins de 13 atomes de carbone, en particulier le mannitol, le xylitol, le sorbitol, le maltitol, les celluloses microcristallines, les sucres et dérivés, le phosphate dicalcique et ses dérivés, la glycine et autres acides aminés pharmaceutiquement compatibles, et leurs dérivés, le lactose et ses dérivés, et leurs mélanges.

L'agent de désagrégation est choisi dans le groupe comprenant notamment la carboxyméthylcellulose sodique réticulée désignée dans le métier par le terme croscarmellose, la crospovidone, le carboxyméthylamidon et leurs mélanges.

L'édulcorant entrant dans la constitution des grains d'excipients est choisi dans le groupe comprenant notamment l'aspartam, l'acesulfame de potassium, le saccharinate de sodium, la néohespéridine dihydrochalcone, le sucralose et leurs mélanges.

L'agent d'écoulement est choisi dans le groupe comprenant notamment la silice colloïdale hydrophobe connue sous le nom de marque Aerosil® R 972

Le liant entrant dans la constitution des grains d'excipients est choisi dans le groupe comprenant notamment l'acide alginique, l'alginate de sodium, l'amidon, l'amidon prégélatinisé, la carboxyméthylcellulose, la dextrine, la gélatine, le sirop de glucose, la gomme guar, les huiles végétales hydrogénées, l'hydroxyéthylcellulose, l'hydroxypropylcellulose, l'hydroxypropylméthylcellulose, le silicate de magnésium et d'aluminium, les maltodextrines, la méthylcellulose, l'oxyde de polyéthylène, les polyméthacrylates, la povidone, la copovidone les polyéthylène glycols, les celluloses microcristallines, les sucres et leurs dérivés, et leurs mélanges.

Selon un mode de réalisation avantageux de l'invention, le liant est choisi dans le groupe comprenant l'acide alginique, l'alginate de sodium, la carboxyméthylcellulose, l'hydroxypropylcellulose faiblement substituée, le silicate de magnésium et d'aluminium, la méthylcellulose, l'amidon, l'amidon prégélatinisé et leurs mélanges.

De façon particulièrement avantageuse, le liant est choisi dans le groupe comprenant l'amidon de maïs, l'amidon prégélatinisé, l'hydroxypropylcellulose, les maltodextrines et leurs mélanges.

L'agent perméabilisant pouvant entrer dans la constitution des grains d'excipients est choisi dans le groupe des silices ayant une grande affinité pour les solvants aqueux, telles que la silice précipitée connue sous le nom de marque Syloïd® ou la silice colloïdale connue sous le nom de marque Aerosil®, les maltodextrines, les β-cyclodextrines, le polyoxyéthylèneglycol micronisé et ses dérivés, et leurs mélanges.

Selon un mode de réalisation avantageux de l'invention, l'agent perméabilisant est la silice précipitée connue sous le nom de marque Syloïd® FP 244.

L'agent aromatisant et le colorant pouvant entrer dans la constitution des grains d'excipients sont choisis parmi ceux qui sont pharmaceutiquement acceptables. Ils sont choisis selon les caractéristiques organoleptiques souhaitées pour le produit final et de façon à masquer au mieux le goût résiduel de la matière active.

L'agent gonflant pouvant entrer dans la constitution des grains d'excipients est choisi dans le groupe comprenant notamment l'amidon, un amidon modifié ou la cellulose microcristalline.

L'agent lubrifiant pouvant entrer dans la constitution des grains d'excipients est choisi dans le groupe comprenant notamment l'acide stéarique, le stéarate de magnésium, le sodium stéaryl fumarate, le polyoxyéthylèneglycol micronisé (Macrogol 6000 micronisé), la leukine, le sodium benzoate et leurs mélanges.

Selon un autre mode de réalisation de l'invention, l'agent lubrifiant peut également être présent ou être exclusivement présent à la surface du comprimé final.

L'agent aromatisant peut être liquide ou solide. Il peut être combiné à des exhausteurs de goût, tel que l'acide citrique, des agents rafraîchissants tels que le menthyl lactate et ses dérivés, et tout autre excipient permettant l'obtention de qualités gustatives satisfaisantes.

Selon différents modes d'introduction, l'arôme et le colorant peuvent être introduits, soit lors de la fabrication du granulé d'excipient, soit après la fabrication de ce granulé par opération de mélange.

Selon un mode de réalisation avantageux, l'arôme est introduit après la fabrication du granulé d'excipient, par mélange avec ce dernier.

Les comprimés selon l'invention sont adaptés pour la mise en oeuvre de tout type de matière active se présentant sous forme de microcristaux ou pouvant être granulée.

La matière active peut être choisie dans le groupe comprenant les sédatifs gastro-intestinaux, les antiacides, les antalgiques, les anti-inflammatoires, les vasodilatateurs coronariens, les vasodilatateurs périphériques et cérébraux, les anti-infectieux, les antibiotiques, les antiviraux, les antiparasitaires, les anticancéreux, les anxiolytiques, les neuroleptiques, les stimulants du système nerveux central, les antidépresseurs, les antihistaminiques, les antidiarrhéiques, les laxatifs, les suppléments nutritionnels, les immunodépresseurs, les hypocholestérolémiants, les hormones, les enzymes, les antispasmodiques, les antiangoreux, les médicaments influençant le rythme cardiaque, les médicaments utilisés dans le traitement de l'hypertension artérielle, les antimigraineux, les médicaments influençant la coagulabilité sanguine, les antiépileptiques, les myorelaxants, les médicaments utilisés dans le traitement du diabète, les médicaments utilisés dans le traitement des dysfonctions thyroïdiennes, les diurétiques, les anorexigènes, les antiasthmatiques, les expectorants, les antitussifs, les mucorégulateurs, les décongestionnants, les hypnotiques, les antinauséeux, les hématopoïétiques, les uricosuriques, les extraits de végétaux, les agents de contraste.

Les comprimés conformes à l'invention sont particulièrement adaptés aux matières actives utiles dans les traitements destinés aux enfants ou aux personnes âgées compte tenu de leur facilité de prise.

La matière active est présente dans le comprimé sous forme de microcristaux ou microgranules enrobés.

La taille des particules de matière active varie entre 10 et 30 µm.

L'enrobage des microcristaux ou microgranules de matière active peut être l'un de ceux décrits dans les demandes de brevet FR9109245, FR9704234 et FR9806384.

La composition de la couche fonctionnelle d'enrobage est choisie en fonction des caractéristiques de masquage de goût et/ou de libération de matière active souhaitées.

Selon un mode de réalisation avantageux les comprimés de l'invention sont tels que la dimension moyenne des microcristaux ou microgranules enrobés de matière active est de 100µm à 500µm , de préférence de 200µm à 400µm et la dimension des grains d'excipients est de 70µm à 650µm , de préférence de 180µm à 440µm .

Les comprimés conformes à l'invention présentent une abrasion très faible, qui est inférieure à 2% telle que mesurée selon le protocole décrit dans la pharmacopée française (Xème édition, "V.5.1- abrasion des comprimés", janvier 1993).

Cette abrasion très faible permet d'utiliser des méthodes industrielles classiques de transfert et de conditionnement des comprimés ne nécessitant pas de précautions particulières et permettant une grande rapidité d'exécution. Par ailleurs, le conditionnement retenu peut être un conditionnement classique de comprimés sensibles à l'humidité.

Selon un mode de réalisation avantageux, la composition pondérale des comprimés conformes à l'invention est la suivante:
- 30% à 50%, de préférence 35 à 45% de microcristaux ou microgranules enrobés de matière active;
- 50% à 70%, de préférence 55 à 65% de grains d'excipients;
- 0 à 10%, de préférence moins de 5% d'agent aromatisant;
- et 0 à 6%, de préférence moins de 2% de lubrifiant réparti à la surface du comprimé.

La présente invention porte également sur le procédé de préparation de ces comprimés.

Le procédé consiste en les étapes suivantes :
- préparation des microcristaux ou microgranules enrobés,
- préparation des grains d'excipients,
- éventuellement, addition d'au moins un arôme et/ou au moins un colorant, sur le mélange de grains d'excipients,
- mélange des microcristaux ou microgranules enrobés de matière active et des grains d'excipients,
- et compression du mélange.

Les microcristaux ou microgranules enrobés de matière active peuvent être préparés comme décrit dans les demandes de brevet FR9109245 et FR0014803.

Les grains d'excipients sont préparés par granulation humide du mélange d'excipients.

Selon un mode de réalisation avantageux, l'eau est utilisée comme agent mouillant pour la granulation.

L'étape finale de compression est facile à réaliser étant donné que le mélange des microcristaux ou microgranules enrobés et des grains d'excipients est très homogène, il n'y a donc pas de démélange.

Les comprimés obtenus présentent une grande homogénéité entre eux et une grande homogénéité de la répartition des excipients et donc une abrasion très faible.

Par ailleurs, un avantage supplémentaire est obtenu par la mise en oeuvre de la présente invention. La compression finale étant très aisée, elle peut être réalisée par des agents non spécialisés. Ainsi, il est possible d'acheminer séparément les microcristaux ou microgranules enrobés de matière active et les grains d'excipients et de procéder à la compression sur le lieu de la commercialisation. Ceci afin de diminuer les problèmes d'acheminement de produits très sensibles à l'eau.

L'invention pourra être mieux comprise à l'aide des exemples qui suivent et qui ne sont pas limitatifs mais relatifs à des modes de réalisation avantageux de l'invention.

### EXEMPLES

### Exemple 1 : Préparation de grains d'excipients

Dans un mélangeur, on introduit 4866g de Mannitol 60, 850g de Kollidon CL, 284g d'aspartam et 300g de L-HPC LH 21. On homogénéise pendant 5 minutes ce mélange puis on mouille ce mélange avec 2,51 d'eau pendant 1 minutes. Le mélange obtenu est versé dans un appareil de démottage puis dans un granulateur à grilles de 1,5mm, de type Alexanderwerk. Le mélange est ensuite séché dans un sécheur à lit d'air de type Glatt GPCG3. Les grains obtenus sont alors tamisés sur grille de 0,8mm.

La répartition granulométrique des grains d'excipients ainsi préparés est comprise entre 80µm et 610µm.

La granulométrie est centrée sur 450 µm.

Les grains sont ensuite imprégnés d'arôme banane liquide.

### Exemple 2: Préparation de comprimés de paracétamol.

On introduit les microcristaux de paracétamol dans une installation à lit d'air fluidisé et on pulvérise sur les microcristaux une dispersion dans l'éthanol d'EUDRAGIT E 100, d'EUDRAGIT NE 30 D et de silice colloïdale de façon à obtenir des microcristaux enrobés avec 10% de polymère.

La répartition granulométrique des microcristaux enrobés ainsi obtenus est de 290-619µm avec un pic à 440µm.

On mélange alors les microcristaux enrobés de paracétamol ainsi obtenus avec des grains d'excipients tels que préparés dans l'exemple 1, dans une proportion en poids de 65% de grains d'excipients pour 35% de microgranules enrobées de paracétamol.

Le mélange ainsi obtenu est comprimé sur une comprimeuse de type Fette à l'aide de poinçons de 12 à une force de compression de 9,5KN.

La dureté des comprimés obtenus est de 35 à 40N. Cette dureté a été mesurée à l'aide d'un appareil de mesure de résistante à la rupture de type Erweka.

Le temps de désagrégation en bouche est de 10 secondes environ. Ce temps correspond à la durée qui sépare, d'une part, le moment de la mise en place du comprimé dans la bouche au contact de la salive et, d'autre part, le moment de la déglutition de la suspension résultant de la désagrégation du comprimé au contact de la salive.

L' abrasion est de 0,4%. L' abrasion est mesurée selon le protocole décrit dans la pharmacopée française (Xème édition, "V.5.1- abrasion des comprimés", janvier 1993).

## Revendications

1. Comprimé à délitement rapide du genre de ceux qui sont destinés à se désintégrer dans la bouche au contact de la salive en moins de 40 secondes, de préférence en moins de 30 secondes, et en formant une suspension facile à avaler, qui est à base d'au moins une matière active sous forme de microcristaux ou de microgranules enrobés et d'un mélange d'excipients sous forme de grains, **caractérisé en ce que** le mélange d'excipients comprend:
de 60 à 85% d'un diluant,
de 3% à 20% d'un agent de désagrégation,
de 1% à 8% d'un édulcorant,
de 0% à 5% d'un agent d'écoulement,
de 0% à 10% d'un liant,
de 0% à 10% d'un agent perméabilisant, d'un agent gonflant et/ou d'un agent lubrifiant,
les pourcentages étant des pourcentages en poids par rapport au poids total des grains d'excipients,
et par le fait que les grains d'excipients ont une granulométrie médiane comprise entre +30 et -30%, de préférence entre +10 et -10%, par rapport à la dimension des microcristaux ou microgranules enrobés.

2. Comprimé selon la revendication 1, **caractérisé par le fait que** le diluant est choisi dans le groupe comprenant notamment les polyols de moins de 13 atomes de carbone, en particulier le mannitol, le xylitol, le sorbitol, le maltitol, les celluloses microcristallines, les sucres et leurs dérivés, le phosphate dicalcique et ses dérivés, la glycine et autres acides aminés pharmaceutiquement compatibles, et leurs dérivés, le lactose et ses dérivés et leurs mélanges, que l'agent de désagrégation est choisi dans le groupe comprenant notamment la carboxyméthylcellulose sodique réticulée désignée dans le métier par le terme croscarmellose, la crospovidone, le carboxyméthylamidon et leurs mélanges.

3. Comprimé selon la revendication 2, **caractérisé par le fait que** le liant est choisi dans le groupe comprenant notamment l'acide alginique, l'alginate de sodium, l'amidon, l'amidon prégélatinisé, la carboxyméthylcellulose, la dextrine, la gélatine, le sirop de glucose, la gomme guar, les huiles végétales hydrogénées, l'hydroxyéthylcellulose, l'hydroxypropylcellulose, l'hydroxypropylméthylcellulose, le silicate de magnésium et d'aluminium, les maltodextrines, la méthylcellulose, l'oxyde de polyéthylène, les polyméthacrylates, la copovidone, la povidone, les polyéthylène glycols, les celluloses microcristallines, les sucres et leurs dérivés et leurs mélanges.

4. Comprimé selon l'une quelconque des revendications 1 à 3, **caractérisé par le fait que** les grains d'excipients peuvent en outre comprendre un arôme sous forme liquide ou de préférence sous forme pulvérulente ou particulaire, ajouté préférentiellement par mélange après la fabrication du granulé d'excipient proprement dit.

5. Comprimé selon l'une quelconque des revendications 1 à 4, **caractérisé par le fait que** le comprimé peut en outre comprendre un lubrifiant sous forme pulvérulente réparti à la surface du comprimé.

6. Comprimé selon l'une quelconque des revendications 1 à 5, **caractérisé par le fait que** le comprimé peut en outre comprendre au moins un agent aromatisant et/ou au moins un colorant

7. Comprimé selon l'une quelconque des revendications 1 à 6, **caractérisé par le fait que** sa composition pondérale est la suivante:
30% à 50%, de préférence 35 à 45% de microcristaux ou microgranules enrobés de matière active;
50% à 70%, de préférence 55 à 65% de grains d'excipients;
0 à 10%, de préférence moins de 5% d'agent aromatisant;
et 0 à 6%, de préférence moins de 2% de lubrifiant réparti à la surface du comprimé.

8. Comprimé selon l'une quelconque des revendications 1 à 7, **caractérisé par le fait que** la dimension moyenne des microcristaux ou microgranules enrobés de matière active est de 100µm à 500µm , de préférence de 200µm à 400µm et la dimension des grains d'excipients est de 70µm à 650µm , de préférence de 180µm à 440µm .

9. Comprimé selon l'une quelconque des revendications 1 à 8, **caractérisé par le fait qu'**il présente une abrasion, inférieure à 2% mesurée comme indiqué dans la pharmacopée française (Xème édition, "V.5.1- abrasion des comprimés", janvier 1993).

10. Procédé de préparation de comprimé selon l'une quelconque des revendications 1 à 9, **caractérisé par le fait qu'**il comprend les étapes consistant à:
- préparer les microcristaux ou microgranules enrobés de matière active,
- granuler par voie humide le mélange d'excipients;
- éventuellement ajouter un arôme et un colorant aux grains d'excipients;
- mélanger les grains d'excipients ainsi obtenus avec les microcristaux ou microgranules enrobés de matière active ;
- et comprimer à sec le mélange ainsi obtenu.

## Patentansprüche

1. Tablette mit schnellem Zerfall von der Art derer, die dazu bestimmt sind, im Mund bei Kontakt mit Speichel in weniger als 40 Sekunden, vorzugsweise weniger als 30 Sekunden, und unter Bildung einer Suspension, welche einfach zu schlucken ist, zu zerfallen, die als Grundlage mindestens einen Wirkstoff in Form von Mikrokristallen oder Mikrokörnchen, die umhüllt sind, und ein Exzipienzien-Gemisch in Form von Körnern hat, **dadurch gekennzeichnet, daß** das Exzipienzien-Gemisch umfaßt:
- 60 bis 85 % eines Verdünnungsmittels,
- 3 bis 20 % eines Zerfallsmittels,
- 1 bis 8 % eines Süßstoffs,
- 0 bis 5 % eines Verlaufsmittels,
- 0 bis 10 % eines Bindemittels,
- 0 bis 10 % eines Permeabilisierungsmittels, eines Bulkingagenses und/oder eines Gleitmittels,
wobei die Prozentangaben Gewichtsprozente, bezogen auf das Gesamtgewicht der Körner der Exzipienzien, sind, und durch die Tatsache, daß die Körner der Exzipienzien eine mittlere Granulometrie zwischen +30 und -30 %, vorzugsweise zwischen +10 und -10 % haben, bezogen auf die Abmessungen der Mikrokristalle oder Mikrokörnchen, die umhüllt sind.

2. Tablette nach Anspruch 1, **dadurch gekennzeichnet, daß** das Verdünnungsmittel aus der Gruppe, die insbesondere die Polyole mit weniger als 13 Kohlenstoffatomen, insbesondere Mannitol, Xylitol, Sorbitol, Maltitol, mikrokristalline Cellulosen, Zucker und ihre Derivate, Dicalciumphosphat und seine Derivate, Glycin und andere pharmazeutisch kompatible Aminosäuren und ihre Derivate, Lactose und seine Derivate und ihre Gemische umfaßt, ausgewählt ist und daß das Zerfallsmittel aus der Gruppe, die vernetztes Natriumcarboxymethylcellulose auf dem Fachgebiet mit dem Ausdruck Croscarmellose bezeichnet, Crospovidon, Carboxymethylstärke und ihre Gemischen umfaßt, ausgewählt ist.

3. Tablette nach Anspruch 2, **dadurch gekennzeichnet, daß** das Bindemittel aus der Gruppe, die insbesondere Alginsäure, Natriumalginat, Stärke, vorgelatinierte Stärke, Carboxymethylcellulose, Dextrin, Gelatine, Glucosesirup, Guargummi, hydrierte Pflanzenöle, Hydroxyethylcellulose, Hydroxypropylcellulose, Hydroxypropylmethylcellulose, Magnesium- und Aluminiumsilicat, Maltodextrine, Methylcellulose, Polyethylenoxide, Polymethacrylate, Copovidon, Povidon, Polyethylenglykole, mikrokristalline Cellulosen, Zucker und ihre Derivate und ihre Gemische umfaßt, ausgewählt ist.

4. Tablette nach einem der Ansprüche 1 bis 3, **dadurch gekennzeichnet, daß** die Körner der Exzipienzien außerdem ein Aroma in flüssiger Form oder vorzugsweise in pulverförmiger oder partikelförmiger Form umfassen, das vorzugsweise durch Zumischen nach Herstellung des eigentlichen Exzipienzgranulats zugesetzt wird.

5. Tablette nach einem der Ansprüche 1 bis 4, **dadurch gekennzeichnet, daß** die Tablette außerdem ein Gleitmittel in pulverförmiger Form an der Oberfläche der Tablette verteilt umfassen kann.

6. Tablette nach einem der Ansprüche 1 bis 5, **dadurch gekennzeichnet, daß** die Tablette außerdem mindestens ein Aromatisierungsmittel und/oder mindestens ein Farbmittel umfassen kann.

7. Tablette nach einem der Ansprüche 1 bis 6, **dadurch gekennzeichnet, daß** ihre Gewichtszusammensetzung folgende ist:
30 % bis 50 %, vorzugsweise 35 bis 45 % Mikrokristalle oder Mikrokörnchen aus Wirkstoff, die umhüllt sind;
50 % bis 70 %, vorzugsweise 55 bis 65 % Körner von Exzipienzien;
0 bis 10 %, vorzugsweise weniger als 5 % Aromatisierungsmittel und
0 bis 6 %, vorzugsweise weniger als 2 % Gleitmittel, auf der Oberfläche der Tablette verteilt.

8. Tablette nach einem der Ansprüche 1 bis 7, **dadurch gekennzeichnet, daß** die mittlere Größe der Mikrokristalle oder der Mikrokörnchen aus Wirkstoff, die umhüllt sind, 10 µm bis 500 µm, vorzugsweise 200 µm bis 400 µm ist und die Größe der Körner der Exzipienzien 70 µm bis 650 µm, vorzugsweise 180 µm bis 440 µm ist.

9. Tablette nach einem der Ansprüche 1 bis 8, **dadurch gekennzeichnet, daß** sie einen Abrieb, gemessen wie in der französischen Pharmakopöe (10. Ausgabe, "V.5.1-abrasion des comprimés", Januar 1993) angegeben, von unter 2 % aufweist.

10. Verfahren zur Herstellung einer Tablette nach einem der Ansprüche 1 bis 9, **dadurch gekennzeichnet, daß** es die Stufen umfaßt, die bestehen aus:
- Herstellung der Mikrokristalle oder Mikrokörnchen aus Wirkstoff, die umhüllt sind,
- Granulieren des Gemisches der Exzipienzien auf nassem Weg;
- gegebenenfalls Zufügen eines Aromas und eines Farbmittels zu den Körnern der Exzipienzien;
- Mischen der so erhaltenen Körner der Exzipienzien mit den Mikrokristallen oder Mikrokörnchen aus Wirkstoff, die umhüllt sind; und
- Verpressen des so erhaltenen Gemischs in der Trockene.

## Claims

1. Fast release tablet which disintegrates in the mouth upon contact with saliva in less than 40 seconds, preferably in less than 30 seconds, thus resulting in an easy-to-swallow suspension which is based on at least one active substance in the form of coated microcrystals or microgranules and on a mixture of excipients in the form of grains, **characterized in that** the mixture of excipients comprises:
from 60 to 85% of a diluent,
from 3% to 20% of a disintegrant,
from 1% to 8% of a sweetener,
from 0% to 5% of a flow agent,
from 0% to 10% of a binder,
from 0% to 10% of a permeabilizing agent, swelling
agent and/or lubricant,
the percentages being percentages by weight relative to the total weight of the grains of excipients,
and **in that** the grains of excipients have a median particle size of between +30 and -30%, preferably between +10 and -10%, relative to the size of the coated microcrystals or microgranules.

2. The tablet of claim 1, **characterized in that** the diluent is selected from the group comprising in particular polyols with less than 13 carbon atoms, especially mannitol, xylitol, sorbitol, and maltitol, microcrystalline celluloses, sugars and derivatives thereof, dicalcium phosphate and its derivatives, glycine and other pharmaceutically compatible amino acids, and their derivatives, lactose and its derivatives, and mixtures thereof, and **in that** the disintegrant is selected from the group comprising in particular crosslinked sodium carboxymethylcellulose, which is designated in the art by the term croscarmellose, crospovidone, carboxymethylstarch, and mixtures thereof.

3. The tablet of claim 2, **characterized in that** the binder is selected from the group comprising in particular alginic acid, sodium alginate, starch, including pregelatinized starch, carboxymethylcellulose, dextrin, gelatine, glucose syrup, guar gum, hydrogenated vegetable oils, hydroxyethylcellulose, hydroxypropylcellulose, hydroxypropylmethylcellulose, aluminum magnesium silicate, maltodextrins, methylcellulose, polyethylene oxide, polymethacrylates, copovidone, povidone, polyethylene glycols, microcrystalline celluloses, sugars and their derivatives, and mixtures thereof.

4. The tablet of any one of claims 1 to 3, **characterized in that** the grains of excipients may further comprise a flavoring agent in liquid form or, preferably, in pulverulent or particulate form which is preferably added after the manufacture of the excipient granules.

5. The tablet of any one of claims 1 to 4, **characterized in that** the tablet may further comprise a lubricant in pulverulent form which is distributed on the surface of the tablet.

6. The tablet of any one of claims 1 to 5, **characterized in that** the tablet may further comprise at least one flavoring agent and/or at least one colorant.

7. The tablet of any one of claims 1 to 6, **characterized in that** its weight composition is as follows:
30% to 50%, preferably 35 to 45%, of coated microcrystals or microgranules of active substance;
50% to 70%, preferably 55 to 65%, of grains of excipients;
0 to 10%, preferably less than 5%, of flavoring agent;
and 0 to 6%, preferably less than 2%, of lubricant distributed on the surface of the tablet.

8. The tablet of any one of claims 1 to 7, **characterized in that** the average size of the coated microcrystals or microgranules of active substance is from 100 µm to 500 µm, preferably from 200 µm to 400 µm, and the size of the grains of excipients is from 70 µm to 650 µm, preferably from 180 µm to 440 µm.

9. The tablet of any one of claims 1 to 8, **characterized in that** it exhibits an abrasion of less than 2%, measured as indicated in the French pharmacopoeia (Xth edition, "V.5.1- abrasion des comprimés" [tablet abrasion], January 1993).

10. A method of preparing a tablet of any one of claims 1 to 9, **characterized in that** it comprises the following steps:
- preparing the coated microcrystals or microgranules of active substance;
- wet-granulating the mixture of excipients;
- optionally adding a flavoring agent and colorant to the grains of excipients;
- mixing the grains of excipients thus obtained with the coated microcrystals or microgranules of active substance;
- and dry-tableting the mixture thus obtained.
